# EUROPEAN PATENT APPLICATION

(11) **EP 3 471 063 A1**
(43) Date of publication of application: **17.04.2019**
(21) Application number: 17812641.3
(22) Date of filing: 09.06.2017
(51) Int. Cl.: G06T 15/00

(54) **THREE-DIMENSIONAL IMAGING METHOD AND SYSTEM**

(30) Priority: 12.06.2016 CN 201610412100
(71) Applicant: Telefield Medical Imaging Limited, Hong Kong (CN)
(72) Inventor: ZHENG, Yongping, Hong Kong (CN)
(74) Representative: Hellmich, Wolfgang
(86) International application number: PCT/CN2017/087769
(87) International publication number: WO 2017/215530

(57) **Abstract**

A three-dimensional imaging method and system. The method comprising the following steps: acquiring, by means of an orthogonal scan probe, at least two orthogonal two-dimensional images of a scanning target (S1); moving the orthogonal scan probe along a specific spatial direction, and acquiring at least two orthogonal two-dimensional images of the scanning target (S2); comparing the two-dimensional images of a corresponding direction obtained from step S1 and step S2, and obtaining movement information of the orthogonal scan probe along the direction (S3); repeating steps S1 to S3 until the scanning target is completely scanned (S4); and reconstructing, by means of the acquired two-dimensional image and three-dimensional motion information of the probe, a three-dimensional image of the scanning target (S5). The system comprises at least two scan probes (201) for acquiring orthogonal images, a computing unit (202) for analyzing continuous motion information of a two-dimensional imaging probe, and a three-dimensional imaging device for acquiring a three-dimensional image. The method and system simplify a structural assembly of an imaging system and further improves upon an economic feasibility.

## Description

### TECHNICAL FIELD

The present application relates to the field of image processing, and more particularly, relates to a three-dimensional imaging method and system by continuously acquiring two-dimensional images.

### BACKGROUND

Three-dimensional imaging is widely used in the medical field, and one of which is to acquire a three-dimensional image by acquiring continuous two-dimensional images and corresponding spaces. In such three-dimensional imaging systems, the spatial position information is usually acquired by one of the following three methods: 1) using a mechanical scanning device to obtain the position and information of each two-dimensional image; 2) applying a spatial information sensor, such as electromagnetic sensor, optical sensor, etc.; 3) using the intrinsic correlation between successively acquired two-dimensional images and the image size. These three methods have their own advantages and disadvantages. Among them, the third method does not need any device other than the scan probe, so it is the most economical and practical method. However, although the scan probe can achieve the three-dimensional imaging effect without any modification, its three-dimensional scanning range is limited, its scanning speed cannot be fast, and it can just scan in one direction without changing the scanning angle arbitrarily during the scanning process, which defects greatly limit the practicality.

### SUMMARY

Aiming at the shortcomings of the above-mentioned three-dimensional imaging technology, a three-dimensional imaging method and system are proposed, which can extract three-dimensional spatial information by using the image information acquired by the probe itself, thereby realizing three-dimensional imaging of the scanning target.

According to an aspect, a three-dimensional imaging method implemented by using three-dimensional spatial information scanned by a constructed two-dimensional scan probe is provided, comprising following steps:
S1) acquiring at least two orthogonal two-dimensional images at a current position of a scanning target by the scan probe;
S2) moving the scan probe by a specific distance or rotating the scan probe by a specific angle along a specific spatial direction, and acquiring at least two orthogonal two-dimensional images of the scanning target;
S3) comparing two groups of the two-dimensional images of a corresponding direction obtained from step S1 and step S2, and obtaining a movement distance and a rotation angle of the scan probe along the corresponding direction;
S4) repeating steps S1 to S3 until the scanning target is completely scanned;
S5) by means of the acquired two-dimensional images, as well as the movement distance and the rotation angle of each image group relative to a previous image group, reconstructing a three-dimensional image of the scanning target.

In the three-dimensional imaging method of the present application, the two-dimensional image is one of an ultrasonic image, an optical tomography (OCT) image, a photoacoustic image, and a terahertz image.

In the three-dimensional imaging method of the present application, one of the two orthogonal two-dimensional images of the scanning target has a relatively low resolution to obtain the movement distance and rotation angle of the scan probe in a plane corresponding to a clear image, not to obtain a final three-dimensional imaging.

In the three-dimensional imaging method of the present application, the two orthogonal two-dimensional images of the scanning target are different in size.

In the three-dimensional imaging method of the present application, the two orthogonal two-dimensional images of the scanning target are obtained in real time with a speed of at least 25 frames/second to ensure that a movement and rotation of the scan probe in an interval time between the images in two adjacent frames mainly occur in one plane.

In the three-dimensional imaging method of the present application, the rotation angle of the two-dimensional image is obtained by an accelerometer provided on the scan probe.

According to another aspect, a three-dimensional imaging system by means of a multi-directional two-dimensional scanning probe is provided, comprising at least two probes for acquiring orthogonal images, an imaging device for acquiring the orthogonal images, a computing unit for analyzing a continuous movement distance and rotation angle of the two-dimensional image probe, an accelerometer provided on the scan probe for acquiring the rotation angle of the two-dimensional orthogonal images.

In the three-dimensional imaging system by means of a multi-directional two-dimensional scanning probe, the image acquired by the imaging device is one of an ultrasonic image, an optical tomography (OCT) image, a photoacoustic image, and a terahertz image.

In the three-dimensional imaging system by means of a multi-directional two-dimensional scanning probe, the probes for acquiring the orthogonal images are different in size.

The three-dimensional imaging method and system proposed by the present application constructs a three-dimensional image of a scanning target by using a three-dimensional imaging device according to image information and three-dimensional spatial information obtained by the orthogonally constructed scan probe, which not only simplifies a structural assembly of an imaging system and reduces the cost, but also improves an economic feasibility of the imaging system.

### BRIEF DESCRIPTION OF THE DRAWINGS

The present application will be further described below in conjunction with the accompanying drawings and embodiments, in which:
Figure 1 is a schematic diagram showing the layout of the scan probes on two planes according to the present application.
Figure 2 is a schematic diagram showing the formation of the two two-dimensional images by using the two scan probes laying out in Figure 1.
Figure 3 is an explanatory diagram showing the movement of the scanning target in different two-dimensional images.
Figure 4 is an explanatory diagram showing the rotation of the scanning target in different two-dimensional images.
Figure 5 is an explanatory diagram of mutually orthogonal scan probes formed by ultrasonic transducers.
Figure 6 is a schematic diagram of the ultrasonic scan probe transducer array in the prior art.
Figure 7 is a schematic diagram of an ultrasonic transducer array having the orthogonal scan probes of the present application.
Figure 8 is a schematic flow chart of a three-dimensional imaging method according to an embodiment of the present application.
Figure 9 is a schematic diagram of an embodiment of the three-dimensional imaging system proposed by the present application.
Figure 10 is a layout diagram of a transducer array composed of three probes.
Figure 11 is a layout diagram of the present application which using two OCT probes.
Figure 12 is a schematic diagram of the present application showing an orthogonal scanning for acquiring two two-dimensional images by using two OCT probes.
Figure 13 is a schematic diagram of the present application showing an orthogonal scanning for acquiring two two-dimensional images by using one OCT probe.
Figure 14 is a layout diagram of the present application which using a photoacoustic imaging probe (including an ultrasound probe and a laser beam) and an orthogonal ultrasound probe.

### DETAILED DESCRIPTION OF THE PREFERRED EMBODIMENT

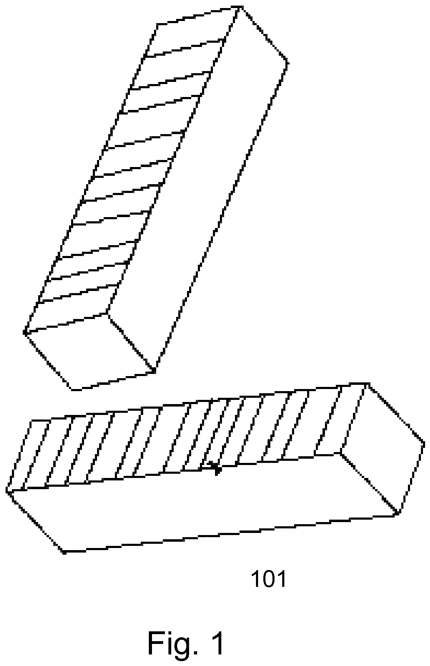

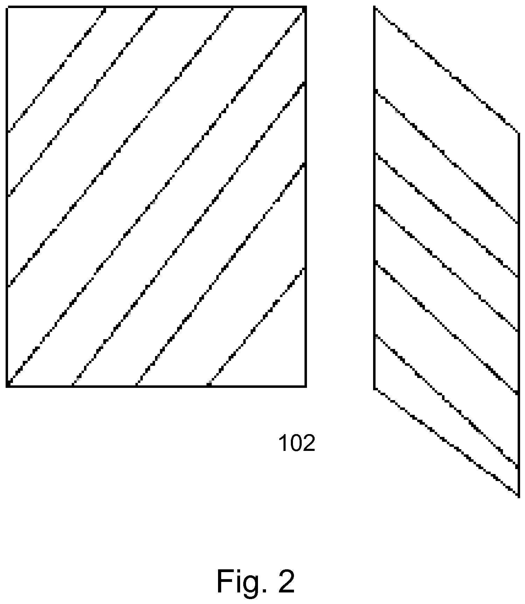

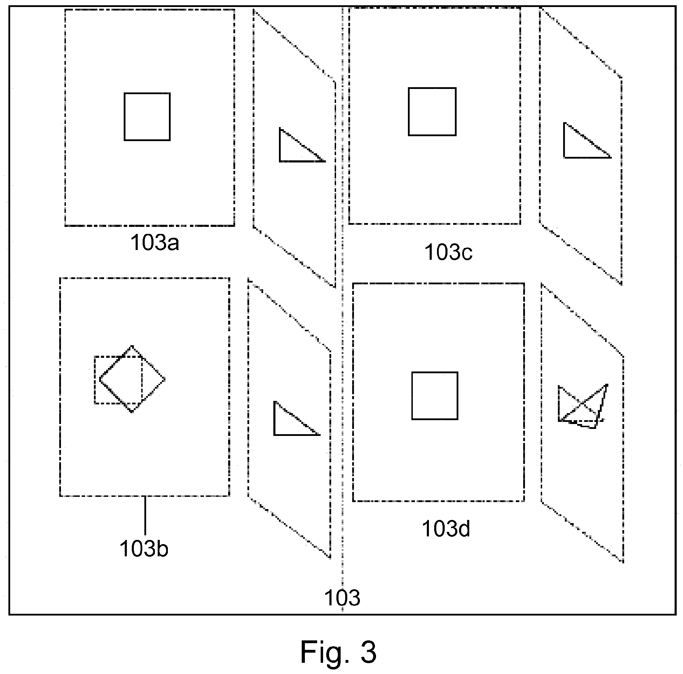

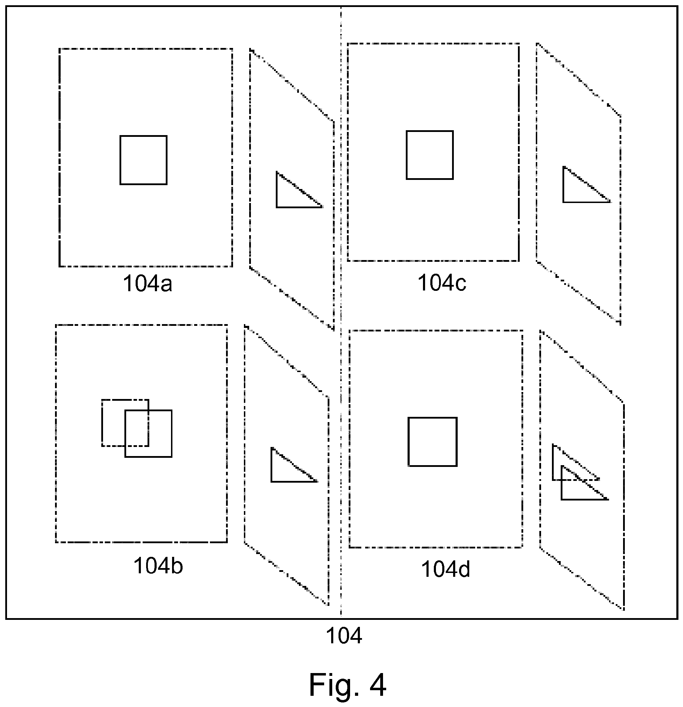

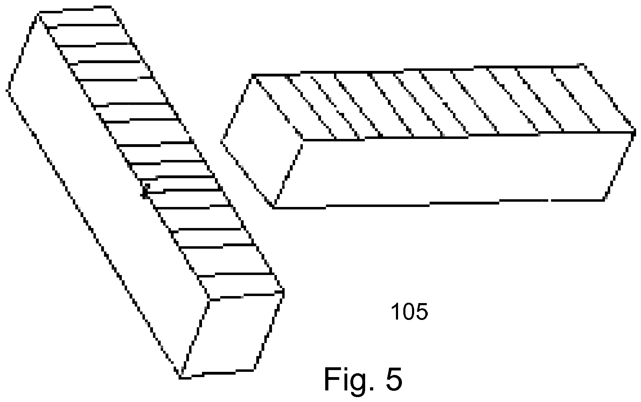

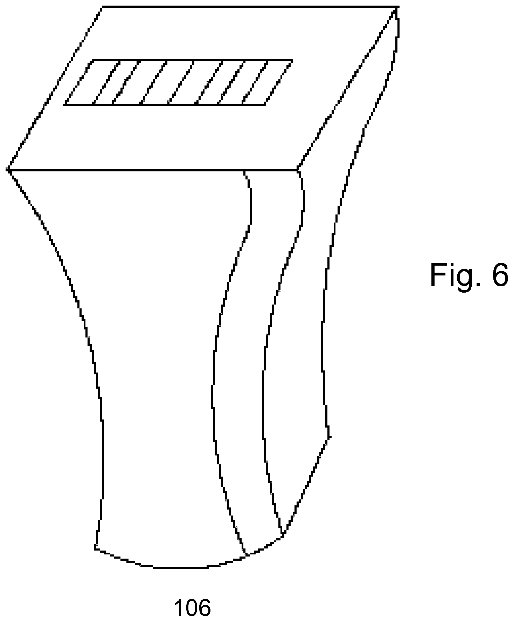

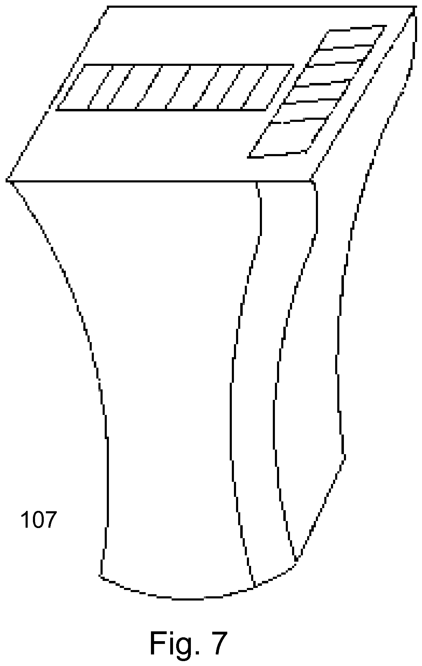

Figure 1 is a schematic diagram showing the layout of the scan probes 101 on two planes. As there is only one probe in the existing scanning technology, so each time there is only one obtained two-dimensional image, which is not helpful for the three-dimensional imaging of the scanning target. Figure 2 is a schematic diagram showing the formation of the two two-dimensional images 102 by using the two scan probes laying out in Figure 1. The two orthogonal probes acquire a group of two mutually orthogonal two-dimensional images by scanning the object in each time. Figure 3 is an explanatory diagram showing the movement of the scanning target in different two-dimensional images 103. A group of orthogonal two-dimensional images that change position relative to the previous group of orthogonal two-dimensional images can be obtained by moving the ultrasound transducer array 105 with orthogonal probes 105. Figure 4 is an explanatory diagram showing the rotation of the scanning target in different two-dimensional images 104. A group of orthogonal two-dimensional images at different angles relative to the previous group of orthogonal two-dimensional images can be obtained by rotating the scan probes 105 having an orthogonal transducer array. Figure 5 is an explanatory diagram of the transducer array in the orthogonal scan probes 105 of the present embodiment. Figure 6 is an explanatory diagram of the ultrasound probe transducer array 106 in the prior art, in which there is only one scan probe and only one two-dimensional image can be obtained in each scanning. Figure 7 is a schematic diagram of a transducer array 107 having the orthogonal scan probes according to the present embodiment. The ultrasound transducer array 107 has two mutually orthogonal scan probes, and a group of two mutually orthogonal two-dimensional images can be obtained by scanning the object in each time. Orthogonal two-dimensional images (104, 105) of different positions are acquired by changing the position of the scan probe 107. Orthogonal two-dimensional images (104, 105) at different locations, as well as distances and locations of adjacent groups are continuously obtained. Then the three-dimensional image of the relative scanning target is acquired by a three-dimensional imaging device.

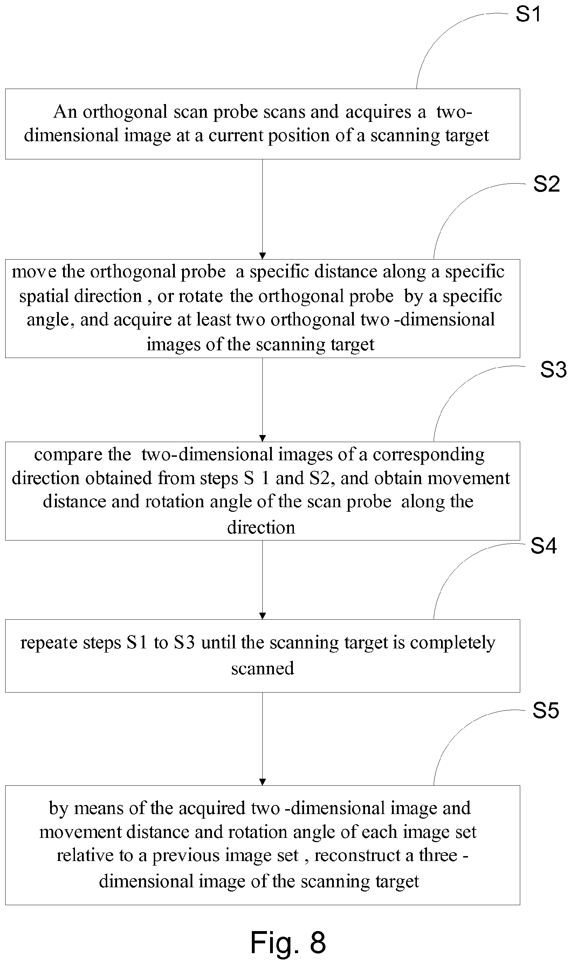

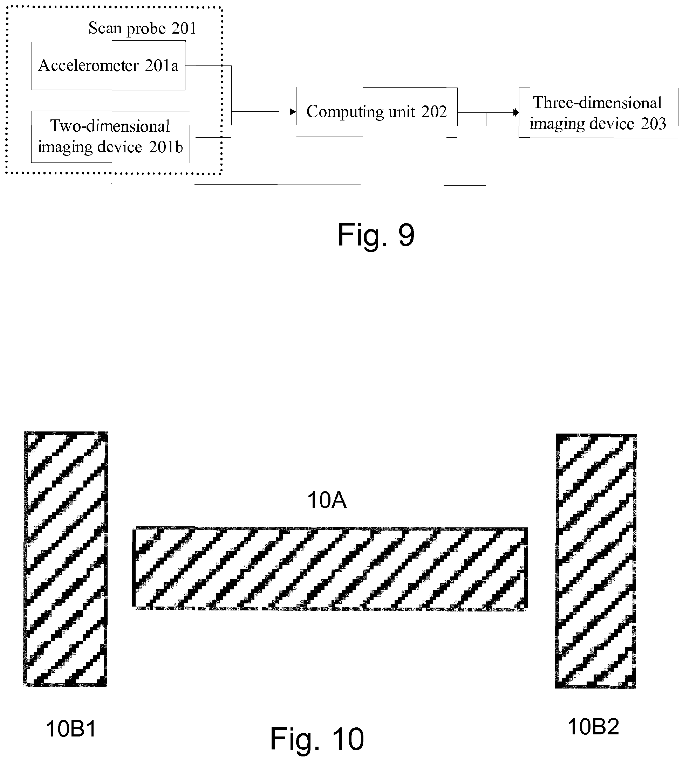

Figure 8 is a schematic flow chart of a three-dimensional imaging method using the spatial information acquired by a scan probe of the present application. Figure 9 is a schematic diagram of a three-dimensional imaging system provided by the present application. Please refer to Figure 1-9, a three-dimensional imaging method using the spatial information acquired by the constructed scan probes comprises the following steps.
S1) During each scanning, the ultrasonic transducer probe 107 constructed by the orthogonal scan probes 105 acquires a group of at least two two-dimensional images (103a, 103c) or (104a, 104c) of the scanning object at the current position. The ultrasonic transducer probe 107 is composed of two orthogonal scan probes. The scanned two-dimensional images 103a and 103c are orthogonal and the two-dimensional images 104a and 104c are orthogonal.
S2) The ultrasonic transducer scan probe 107 is moved by a specific distance or rotated by a specific angle along a specific spatial direction, for acquiring at least two orthogonal two-dimensional images (103b,103d) or (104b,104d) of the scanning target. In order to acquire a three-dimensional image of the scanning target, the ultrasonic transducer scan probe 107 is moved by a specific distance or rotated by a specific angle along a specific spatial direction and simultaneously scans for acquiring a group of orthogonal two-dimensional images (103b,103d) or (104b,104d) after moving or rotating.
S3) The ultrasonic transducer scan probe 107 compares the two groups of orthogonal two-dimensional images (103b,103d) and (103a,103c), or (104b,1034d) and (104a, 104c) of a corresponding direction obtained from steps S1 and S2. The computing unit 202 for calculating the angle and position information of spatial information of different groups of orthogonal images is used to obtain the movement distance and the rotation angle information of the scan probe 105 in the direction.
S4) The steps S1 to S3 are repeated for continuously moving and rotating the ultrasonic transducer probe 107 in a certain direction to scan and store the two-dimensional images of the scanning object after moving and rotating; and sending them to the three-dimensional imaging device 203 for three-dimensional imaging until the scan probe traverses all position information of the scanning target.
S5) After receiving all the two-dimensional images of the scanning target acquired in step S4 and the movement distance and rotation angle information of each image group relative to a previous image group, the three-dimensional imaging device 203 reconstructs the three-dimensional image of the scanning target.

In the present embodiment, referring to Figure 3 or Figure 4, one of the two orthogonal two-dimensional images has a relatively low resolution, and a main purpose of it is to obtain the movement distance and rotation angle of the scan probe 105 in a plane corresponding to a clear image, rather than for obtaining a final three-dimensional imaging of the three-dimensional imaging device 203.

According to the three-dimensional imaging method of the present application, in the present embodiment, referring to Figure 3 or Figure 4, the two orthogonal two-dimensional images of the scanning target are obtained in real time with a speed of at least 25 frames/second to ensure that the movement and rotation of the ultrasonic transducer scan probe 107 in an interval time between the images in two adjacent frames mainly occur in one plane.

According to the three-dimensional imaging method of the present application, in the present embodiment, the rotation angle of two adjacent two-dimensional orthogonal images is obtained by an accelerometer 201a provided on the scan probe 201.

Referring to Figure 9, a three-dimensional imaging system by means of a multi-directional two-dimensional scanning probe according to the present application is provided, which comprises at least two probes 201 for acquiring orthogonal images, a computing unit 202 for analyzing a continuous movement distance and rotation angle of the two-dimensional image probe, an imaging device 203 for acquiring the three-dimensional image.

According to the three-dimensional imaging system of the present application, the scan probe 201 is orthogonally constructed by at least two transducer arrays. On the one hand, the scan probe 201 continuously transmits the orthogonal images scanned in real time to the three-dimensional imaging device 203, and on the other hand transmits the angle information and the distance position information to the computing unit 202. The computing unit 202 transmits the angle information and the distance position information to the three-dimensional imaging device 203 for three-dimensional imaging after comparing and calculating the angle information and the distance position information.

The scan probe 201 comprises a two-dimensional imaging device 201b and an accelerometer for acquiring angle information of the scan probe 201. The two-dimensional imaging device 201b is used for acquiring the two-dimensional orthogonal images, and the accelerometer is used for acquiring the rotation angle information of the scan probe 201.

According to the three-dimensional imaging system 203 of the present application, the acquired image is one of an ultrasonic image, an optical tomography (OCT) image, a photoacoustic image, and a terahertz image.

According to the three-dimensional imaging system 203 of the present application, the scan probes for acquiring orthogonal images are different in size.

Figure 10 is a layout diagram of a transducer array composed of three probes according to the imaging of the present application, in which probes 10B1 and 10B2 are orthogonal to probe10A. The image information formed by the probes 10B1 and 10B2 can complement with each other. If one of them cannot get an image of a good quality, the other image can be used for related calculations. The information obtained in the two images can also be averaged to increase accuracy.

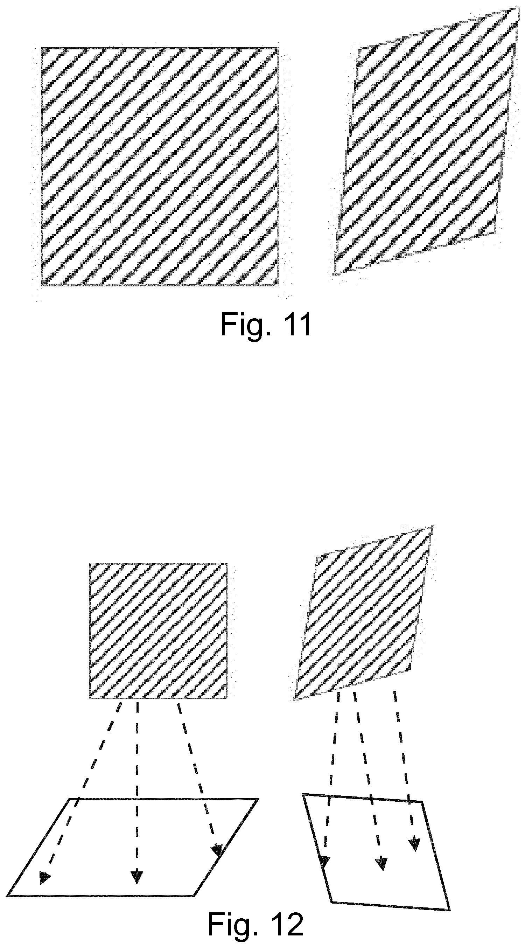

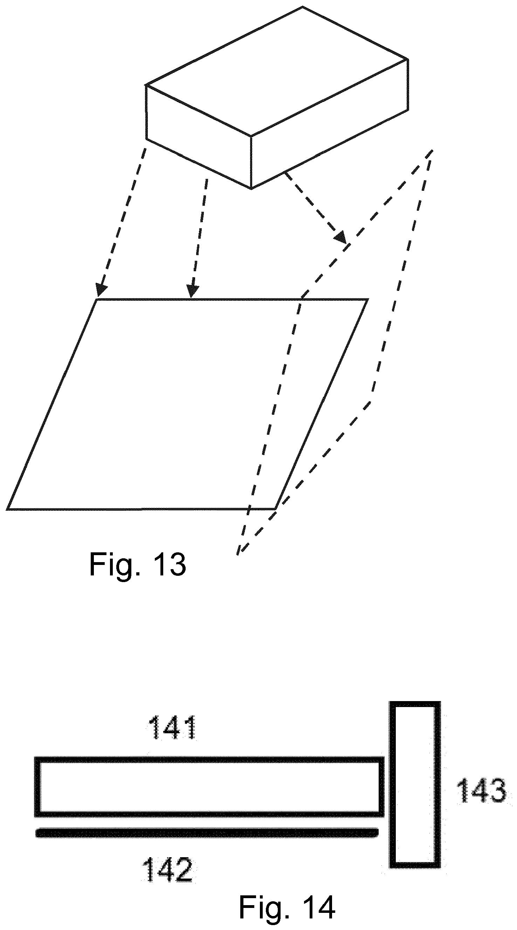

Figure 11 is a layout diagram of the present application which using two OCT probes according to the present application. Figure 12 is a schematic diagram of the present application showing an orthogonal scanning for acquiring two two-dimensional images by using two OCT probes. Figure 13 is a schematic diagram of the present application showing an orthogonal scanning for acquiring two two-dimensional images by using one OCT probe. That is, one OCT probe scans a two-dimensional image at one position, and then scans at an orthogonal position to obtain two orthogonal two-dimensional images.

Figure 14 is a layout diagram of the present application which using a photoacoustic imaging probe (including a main ultrasound probe 141 and a laser beam 142) and an auxiliary orthogonal ultrasound probe 143. The auxiliary ultrasonic transducer array 143 for measuring the movement and rotation information of the scan probe operates in an ultrasound imaging mode, that is, transmitting and receiving ultrasound for imaging, thereby acquiring the movement and rotation of the main ultrasound probe 141 in the corresponding plane. The main ultrasound probe 141 operating in the ultrasound mode can obtain more information measurement of movement and rotation in the corresponding plane, and can also operate in the photoacoustic imaging mode.

The above is only a preferred embodiment of the present application, but the scope of the present application is not limited thereto. Any changes or substitutions that can be easily thought by any person skilled in the art within the technical scope disclosed by the present application are within the scope of the present application. Therefore, the protection scope of the present application should be determined by the scope of the claims.

## Claims

1. A three-dimensional imaging method implemented by using three-dimensional spatial information scanned by a constructed two-dimensional scan probe, **characterised in that** comprising following steps:
S1) acquiring at least two orthogonal two-dimensional images (102) at a current position of a scanning target by a scan probe (107);
S2) moving the scan probe (107) by a specific distance or rotating the scan probe (107) by a specific angle along a specific spatial direction, and acquiring at least two orthogonal two-dimensional images (103b,103d) or (104b,104d) of the scanning target;
S3) comparing two groups of the orthogonal two-dimensional images (103b,103d) and (103a,103c) or (104b,1034d) and (104a,104c) of a corresponding direction obtained from steps S1 and S2, and obtaining movement distance information and rotation angle information of the scan probe (107) along the direction;
S4) repeating steps S1 to S3 until the scanning target is completely scanned;
S5) by means of the acquired two-dimensional images, as well as the movement distance and the rotation angle of each image group relative to a previous image group, reconstructing a three-dimensional image of the scanning target.

2. The three-dimensional imaging method according to claim 1, **characterised in that** the two-dimensional image (103, 104) is one of an ultrasonic image, an optical tomography (OCT) image, a photoacoustic image, and a terahertz image.

3. The three-dimensional imaging method according to claim 1, **characterised in that** one of the two orthogonal two-dimensional images (103,104) of the scanning target has a relatively low resolution to obtain the movement distance and rotation angle of the scan probe in a plane corresponding to a clear image, but not obtain a final three-dimensional imaging.

4. The three-dimensional imaging method according to claim 1, **characterised in that** the two orthogonal two-dimensional images of the scanning target are different in size.

5. The three-dimensional imaging method according to claim 1, **characterised in that** the two orthogonal two-dimensional images (102) or (103,104) of the scanning target are obtained in real time with a speed of at least 25 frames/second to ensure that a movement and a rotation of the scan probe (107) in an interval time between the images in two adjacent frames mainly occur in one plane.

6. The three-dimensional imaging method according to claim 1, **characterised in that** the rotation angle of the two-dimensional image is obtained by an accelerometer (201a) provided on the scan probe (201).

7. A three-dimensional imaging system implemented by continuous two-dimensional images, position information and angle information obtained by orthogonal probes, **characterised in that** comprising at least two scan probes (201) for acquiring orthogonal images, a computing unit (202) for analyzing a continuous movement distance and rotation angle of a two-dimensional image probe, and a three-dimensional imaging device (203) for acquiring a three-dimensional image.

8. The three-dimensional imaging system according to claim 7, **characterised in that** the scan probe (201) is constituted by at least two orthogonal probes; the scan probe (201) continuously sends the orthogonal images scanned in real time to the three-dimensional imaging device (203) and sends the angle information and distance and position information to the computing unit (202); the computing unit (202) sends compared angle information, as well as compared distance and position information to the three-dimensional imaging device (203) for three-dimensional imaging.

9. The three-dimensional imaging system according to claim 7, **characterised in that** the scan probe (201) comprises a two-dimensional imaging device (201b) and an accelerometer (201a) for acquiring the angle information of the scan probe (201); the two-dimensional imaging device (201b) is used for acquiring the two-dimensional orthogonal images; the accelerometer (201a) is used for acquiring the rotation angle information of the scan probe (201).

10. The three-dimensional imaging system according to claim 7, **characterised in that** the probes constituting the scan probe (201) for acquiring the orthogonal images are different in size.
